# EUROPEAN PATENT SPECIFICATION

(11) **EP 1 492 062 B1**
(45) Date of publication and mention of the grant of the patent: **01.05.2013**
(21) Application number: 04013863.8
(22) Date of filing: 14.06.2004
(51) Int. Cl.: G07F 11/42, A47F 1/00

(54) **Integrated programmable system for identification and automatic management of pharmaceutical packets**
Integriertes Programmierbares System für die Identifizierung und für das Automatische Management von den Medikamenten
Système intégré programmable pour l'identification et la gestion automatisée des confections pharmaceutiques

(30) Priority: 27.06.2003 IT TN20030011
(43) Date of publication of application: 29.12.2004
(73) Proprietor: SPID S.P.A., 38123 Trento (IT); BUSTER AUTOMATION S.R.L., 38068 Rovereto, (TN) (IT)
(72) Inventor: Manfredi, Fabrizio, 38068 Rovereto (TN) (IT); Daldin, Ivo, 38100 Trento (IT); Zanotelli, Alessandro, 38100 Trento (IT)
(74) Representative: Modiano, Micaela Nadia

(56) References cited:
- WO-A-00/34925
- WO-A-01/96142
- US-A- 3 189 217
- US-A- 3 716 147
- US-A- 3 884 386
- US-A- 4 896 024
- US-B1- 6 367 653

## Description

This document contains a technical-functional description of a system comprising a cabinet automated by programmable software, which is able to manage logistic processes applied to pharmaceutical packets. The solution presented in this document has been devised and structured to be implemented within hospitals and other medical institutions, such as, for example, hospices or old age homes, chemists and generally all environments which have an interest in automating the flow of stocking, storing and distribution of pharmaceuticals.

In order to achieve this solution, lengthy and thorough research has been carried out, both in Trentino and outside the province, to determine the true needs of these organisations.

### 1) The Situation

Having analysed the demands of environments that are managing logistic packets (hospitals and rest homes etc), it appears that an organisational shortcoming exists in processes connected with the phases of provisioning, loading, stocking, control of stock and subsequent distribution of these products.

SPID and MGT have considered interesting the development of an advanced system that is able to manage the logistic flow of the aforementioned packets, simply and automatically. The research has shown that, at present, personnel responsible for hospital dispensaries execute the following operations manually:
✔ Compilation of weekly requests for replenishment;
✔ Processing of requests;
✔ Controlling of goods at reception;
✔ Loading of the cabinet where the packets are stored;
✔ Control of supplies;
✔ Manual calculation of hourly/daily needs;
✔ Retrieval, from the cabinet, of packets required for the preparation of therapy;
✔ Manual control of pharmaceutical expiry dates;

These activities not only waste highly specialised human resources, but exacerbate the following:
✔ Repeated actions (often cause of frustration);
✔ Uncertainty about stock and availability of materials;
✔ Difficulties connected with the control of expiry dates;
✔ Possible theft of pharmaceuticals;
✔ Difficulties in interchanging trained personnel;
✔ Difficulties in compilation of reliable statistics and as well as difficulties in the control of consumption;

The system presented in this document is able to automatically perform all functions listed previously, thereby eliminating these inconveniences.

WO 00/34925 A discloses a storage and dispensing system for storing and dispensing pharmaceutical products comprising a plurality of product conveyors mounted within a cabinet, an infeed conveyor for transporting the products one by one into the cabinet, an outfeed conveyor for transporting products one by one out of the cabinet, a transporter movable within the cabinet for transporting products between the product conveyors and the infeed and outfeed conveyors. Moreover, an opening, formed in the left side wall of the dispenser, permits the left sides of the infeed conveyor and outfeed conveyor to extend therethrough.

WO 01/96142 A discloses an article dispensing apparatus embodied, for example, as a vending machine, including a controllably positioned suction hose dispenser for retrieving articles from a storage area.

US-B-6 367 653 discloses a centralized method for selling items through a plurality of vending machines installed at various companies' sites comprises using vending machines having a substitutable goods-holding magazines from which goods can be extracted only when fully installed inside a vending machine and only by use of a special key when the magazines are outside their machines. The machine includes a sliding curtain than can be activated by an electric mechanism.

US-A-3 884 386 discloses a device for supplying drinks, others for foodstuffs comprising an electrical closure for the storing cabinet.

### 2) The Solution

The present invention relates to an automatic dispensing unit according to claim 1.

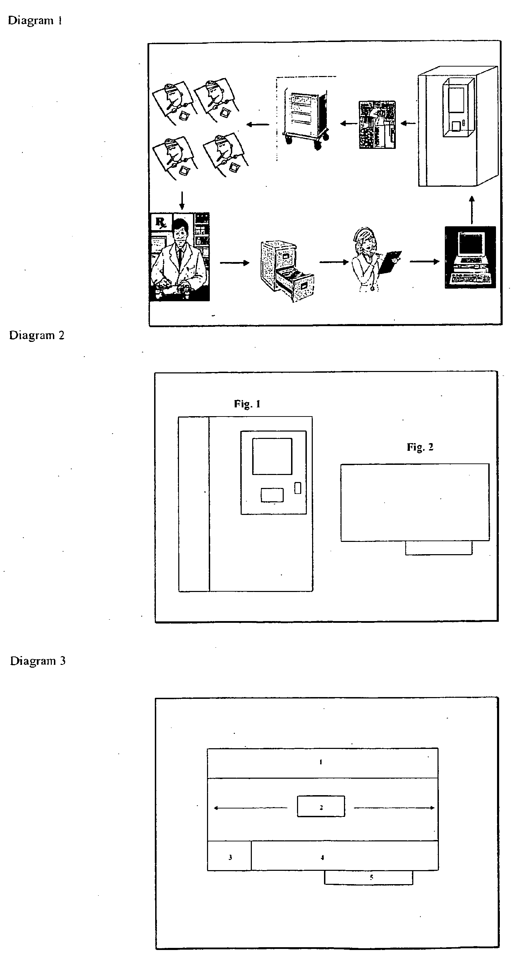

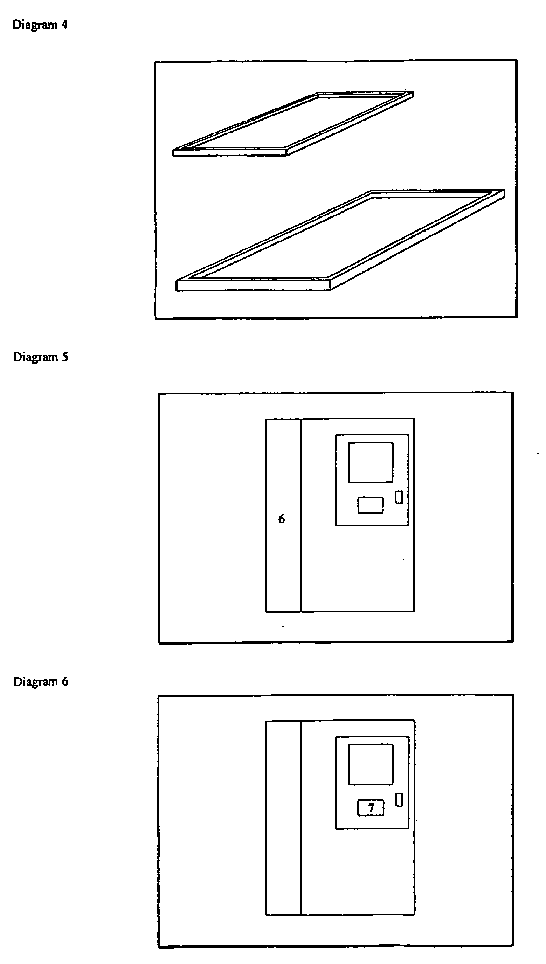

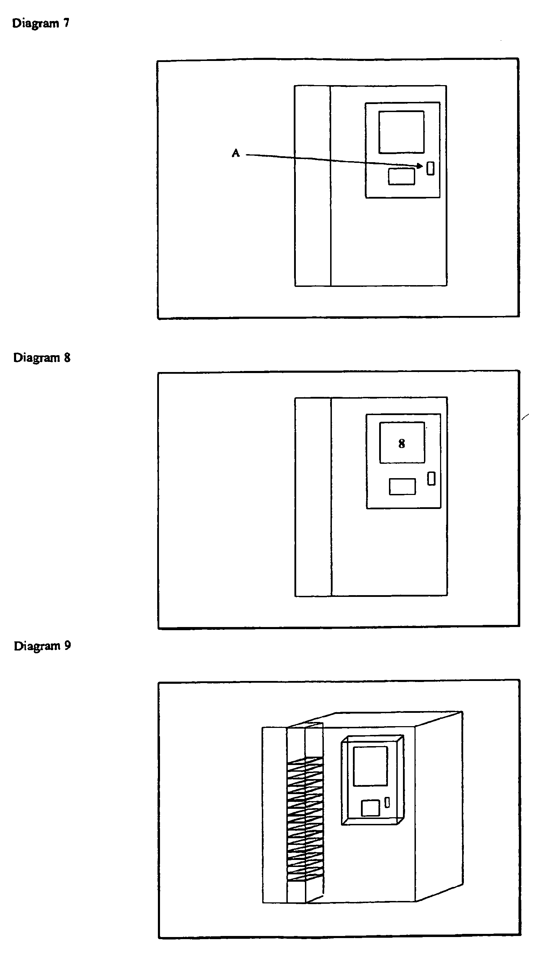

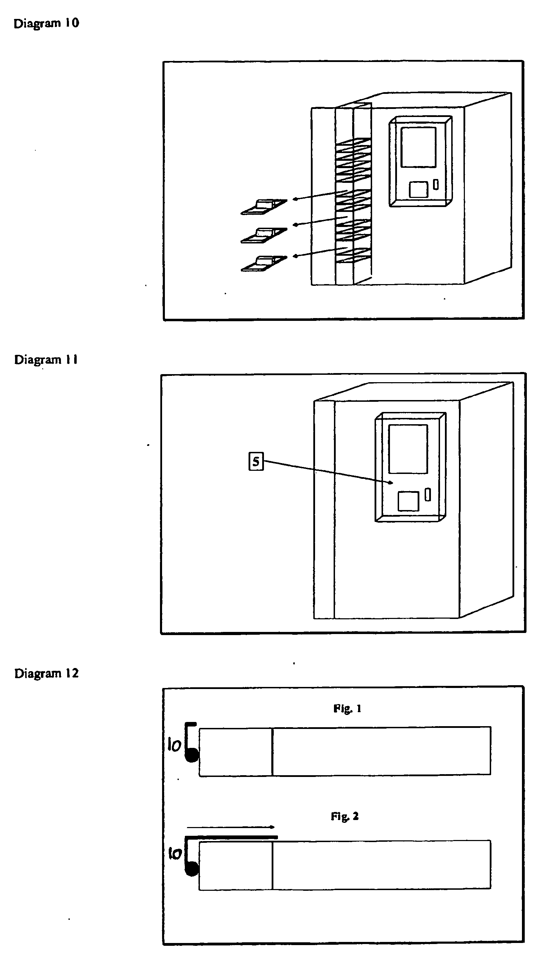

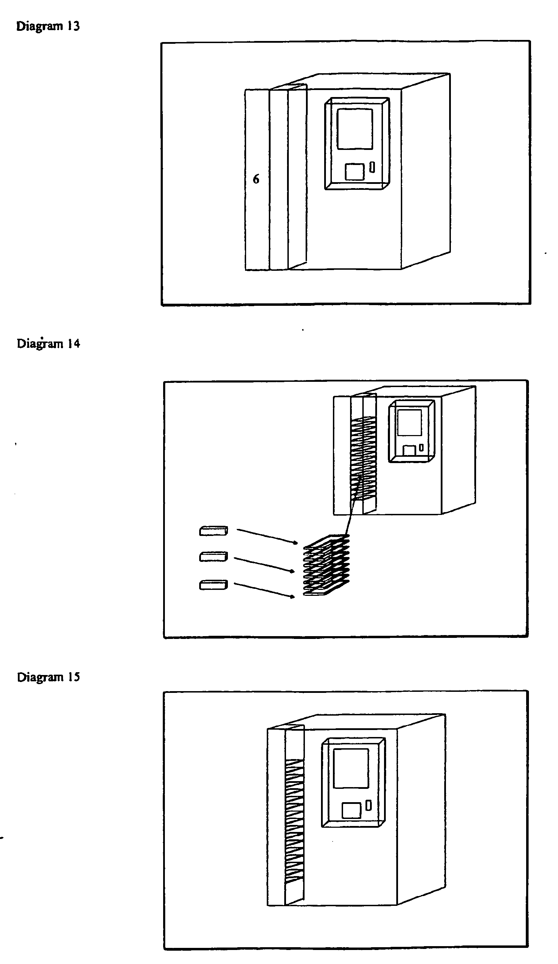

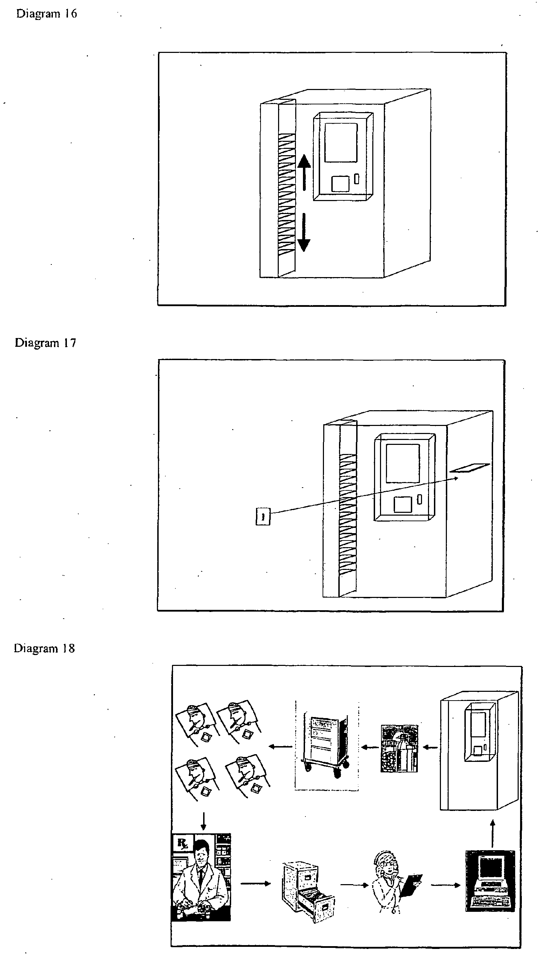

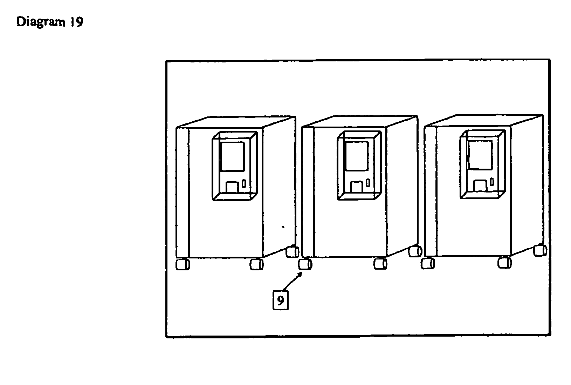

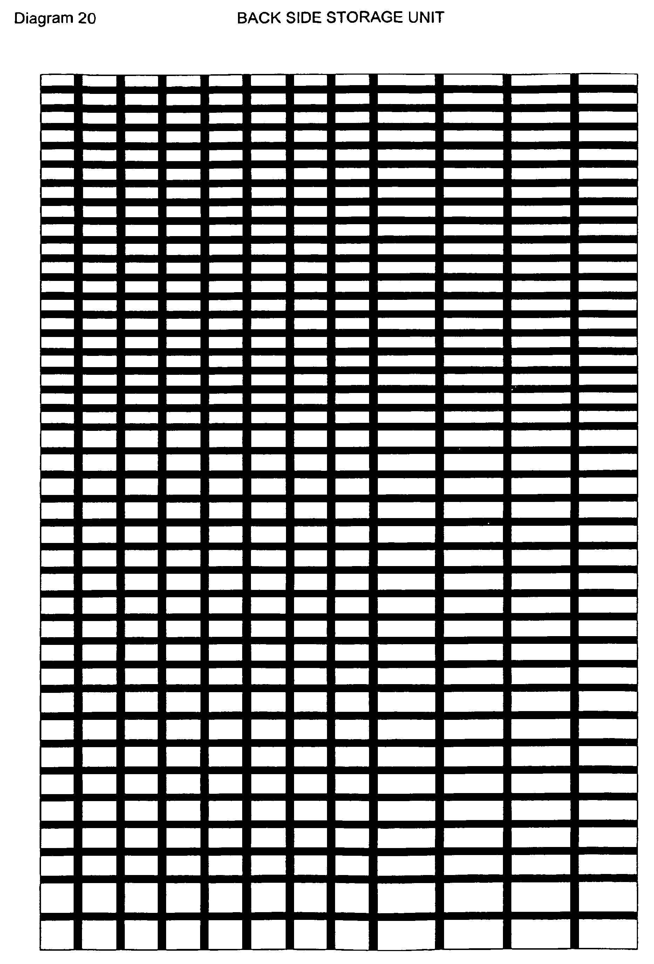

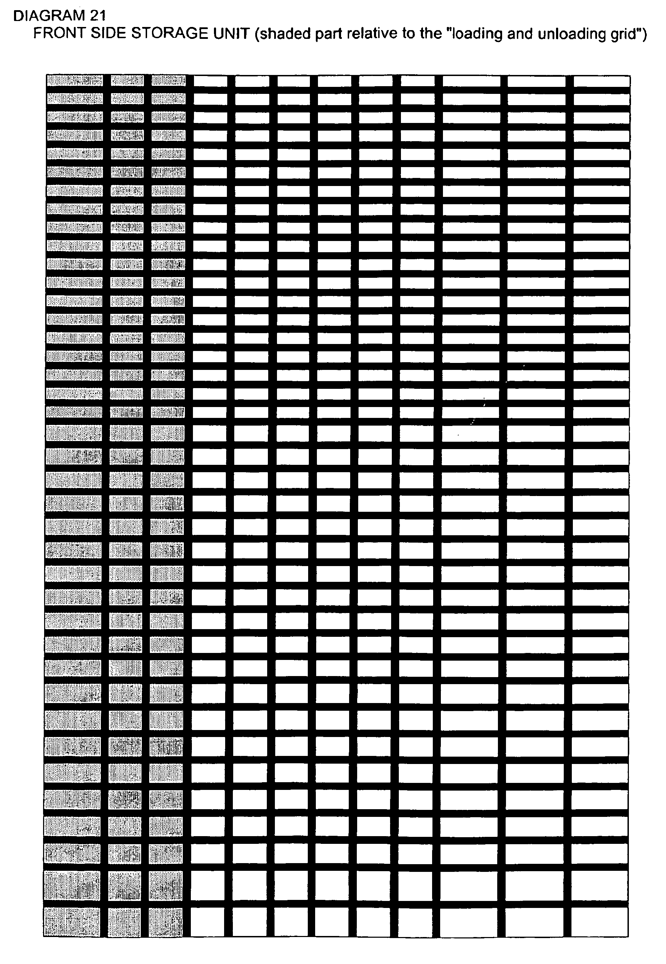

The system consists of an automatic dispenser cabinet which, through the use of particular hardware components, and integrated with specific modules of software, is able to manage the logistic flow of pharmaceutical packets used in medical institutions of various natures. The dispenser can be configured according to the needs of the user. The standard measure, however, is about 200cm in height and 150cm in width (Diagram 2 - Fig.1), by 80 cm in depth (Diagram 2 - Fig.2): the number of objects that can be contained is proportional to the dimensions of the objects themselves.

In the inside of the dispenser there are two storage units which are symmetrical and opposed (Item 1 and Item 4 of Diagram 3 which shows the view of the top of the dispenser). A particular mechanical pincer (Item 2 of Diagram 3) is able to reach every area of the storage unit in order to position the packets managed by the dispenser. The storage units are structured with cells of various dimensions (Diagram 20 and Diagram 21) into which the mechanical pincer inserts particular trays (Diagram 4) containing the pharmaceutical packets. An innovative feature of the system is the fact that a part of the front storage unit (see plan view: Item 3 of Diagram 3; see front view: shaded area of Diagram 21), is open both to the outside and the inside. This area called the "loading and unloading grid", functions as an area of exchange, permitting the loading and unloading of approximately 110 trays (with related packets) for each cycle. A metal door (Item 6 Diagram 5), operated by the distributor through an appropriate electric lock, permits access to the "loading and unloading grid". The system is provided with another point of withdrawal, called "Single Point" (Item 7 - Diagram 6), which permits withdrawal of one packet at a time during the phases when the main "loading and unloading grid" is temporarily occupied (for example, the phase of loading of the goods). Furthermore, the "Single Point" functions as an emergency dispenser in case of mechanical stoppage of the main "loading and unloading grid".

The operator can only interact with the dispenser after positive recognition by the system. Such recognition takes place through the reading of the operator's fingerprints (Item A - Diagram 7) or through input of an identifying code and related password. Once recognised and authorised by the system the operator can execute the various operations available through the use of an appropriate touch-screen monitor (Item 8 - Diagram 8), that provides an extremely user-friendly graphical interface.

According to the profile associated with the fingerprint or identifying code, the operator can either have access to standard withdrawal operations or access a series of particular operations reserved for specialised personnel (supervisors).

The standard procedure of withdrawal is very simple. In the first place the operator proceeds through recognition. Once authorised, the operator compiles through the touch-screen monitor, a list of desired materials and the dispenser proceeds with dispensing of packets through the "loading and unloading grid" (Diagram 9). At this point the operator only has to open the access door of the "loading and unloading grid" (which has been unlocked by the system) and collect the requested goods on related trays (Diagram 10). Having collected the goods, the operator will only have to close the access door and place the empty trays on an appropriate trolley (or another place).

The touch-screen monitor, the personal computer necessary to control the system, the "Single Point", the fingerprint recognition device, and the supplementary bar code reader (for loading and unloading of products to store outside the dispenser), are all located inside a particular console with a mechanical hinge for rapid access (Item 5 - Diagram 11). Due to this device access for normal operations of maintenance and/or repairs is extremely rapid and easy.

An important feature is constituted by a system for locking and unlocking the "loading and unloading grid" (Diagram 12). This device consists of a sliding curtain which opens (Fig. 1-Diagram 12) and closes (Fig. 2 - Diagram 12) to the inside of the "loading and unloading grid". This is necessary, for instance, when during the phases of withdrawal and insertion, the operator pushes the tray too deeply, which could cause subsequent interference with the mechanical pincer and/or dropping the tray into the dispenser.

The procedure of replenishment, meaning the series of operations to load the dispenser with necessary goods, is not very different from the procedure of withdrawal.

The operator (in this case, the supervisor) proceeds through identification, and by means of the touch-screen monitor, selects the appropriate procedure for loading. The dispenser authorises the opening of the access door to the "loading and unloading grid" (Item 6 - Diagram 13) and communicates to the supervisor to insert the pharmaceutical packets into it. The supervisor places these packets on appropriate trays (Diagram 14) and inserts them into cells in the "loading and unloading grid". At this stage the supervisor will just have to be careful to insert the trays into the cells that are as small as possible, thereby making optimum use of the space inside the dispenser. After loading the trays with related packets, the supervisor closes the door (Diagram 15). The system opens the sliding curtain (Fig. 1 - Diagram 12) and executes a rapid search of present trays (Diagram 16). After determining the number and position of trays, they are collected, identified and placed in empty cells of the storage units (Diagram 17). If the number of cells of the "loading and unloading grid" is not sufficient to load all the goods at one time, the loading cycle is repeated until all the pharmaceutical packets are loaded.

Due to its capacity to memorise all the movements of managed packets, the system is able to execute particular functions of control. One of these important functions consists in the control of expiry dates of pharmaceuticals. The dispenser is able to control the period of storage of pharmaceuticals inside it, generating a warning when one or more products appears to be stored for more than a specified number of days or months (this value is modifiable on the basis of the needs of the user). This feature is of great value as it solves the problems arising from the fact that, for example in Italy, it is not currently possible to determine the expiry date from the information contained in the printed bar code on each packet. Another important function is the rigorous application of the FIFO principle (FIRST IN FIRST OUT), which further reduces the period of storage inside the dispenser.

One of the more innovative characteristics of the system is the ability to match the functionality of the dispenser with the real pharmaceutical requirements of the institution that utilises it. To this aim special software, able to manage the therapy intake of patients in electronic format, has been developed. The software is installed on a personal computer connected by a network to the dispenser. The result is that the system executes precisely and automatically all operations normally associated with management of pharmaceutical packets (Diagram 18). As a consequence of this integration it is possible to:
✔ Calculate the quota of pharmaceuticals on an hourly/daily/weekly basis;
✔ Execute automatic daily and/or hourly withdrawal of materials needed for the preparation of therapies;
✔ Generate and send requests for replenishment (daily, weekly, monthly etc.) to authorised suppliers;
✔ Check the minimum levels and re-ordering levels with consequent reduction of the quantity of pharmaceuticals in storage;
✔ Double-check that goods at reception correspond with the order;
✔ Check in real time the quantity of stock in storage;
✔ Check in real time the situation of supplies from external entities (through remote interrogation) with the aim, for example, of establishing a cost centre.

Furthermore the system is able to:
✔ Relieve personnel of the risk of mistakes and facilitate interchange of staff responsible for managing pharmaceuticals;
✔ Manage maintenance and provisioning by means of outsourcing;
✔ Check consumption and generate accurate statistics, eliminating wastage of materials;
✔ Prevent theft;
✔ Integrate with modules of software for management information;
✔ Be programmed and configured based on the specific characteristics of the end user.

The system is mobile as it is mounted on appropriate trolley wheels (Item 9 - Diagram 19). The dispenser is modular, being suitable for use in batteries and thereby expanding without limit the capacity to load (Diagram 19).

The system, finally, can be remotely controlled by a call centre, able to assure assistance, diagnostics and real time warnings. For emergencies it is also fitted with an autonomous source of power (UPS).

### 1) Characteristics and features of the "loading and unloading grid".

The term "loading and unloading grid" (shaded area of Diagram 21) means an electromechanical apparatus composed of a grid of cells of various widths and lengths, able to accommodate pharmaceutical packets of varying dimensions placed on appropriate trays. The "loading and unloading grid" is supplied with an adequate "mechanical template" of adequate measurements for the correct insertion of the aforementioned trays with associated packets.

The "loading and unloading grid" is fitted with an external battened door (Item 6-Diagram 5) which is served, mechanically and electronically, by an appropriate electric lock and an opening system with a powerful spring. Internally the "loading and unloading grid" is provided with a horizontal sliding curtain, activated by an electric mechanism (Fig. 1 and Fig. 2 - Diagram 12), which permits opening and closing to the inside of the "loading and unloading grid".

The "loading and unloading grid" is optimised so that it can function best by using appropriate trays (Diagram 4). Strictly speaking, the term "tray" means a plastic element 2,5 mm thick, bordered on four sides to a height of 9 mm.

The "loading and unloading grid" is supplied with sensors to indicate "product present".

### 2) Mechanical pincer for presence detection, collection and transport of products.

The mechanical pincer (Item 2 - Diagram 3), necessary for the movement of products is also provided with a front sensor to detect the presence of a tray, which permits scanning from low to high (and vice versa) of the whole "loading and unloading grid". In this way it is possible to rapidly identify an object which has been placed in any of the cells of the "loading and unloading grid". Monitoring detection of the presence of the trays, the pincer withdraws a tray to momentarily position it in the proximity of the active matrix reader. In a matter of seconds the reader is able to identify the pharmaceutical packet by decoding the bar code depicted on it. Thereafter, the tray and related packet are stored in a cell of the internal storage unit by the pincer.

### 3) Active matrix system for reading of bar codes.

For the reading of bar codes of pharmaceutical packets there is an appropriate active matrix reader. The pincer positions the products in the reader area for scanning. The reader reads the entire area of the tray and, detecting the presence of one or more packets, acquires an alpha-numeric string associated with the bar code. Successively the software interrogates the appropriate database, obtaining not only the identification of the products, but also a series of important pieces of additional information.

### 4) Double apparatus for expulsion of products by means of conveyor belt with sliding vertical control gate. (Single Point).

The system enables withdrawal of products both through the main "loading and unloading grid" ("multiple" delivery of up to approximately 110 products) and withdrawal of a single product through an appropriate device called "Single Point" (Item 7 - Diagram 6).

### 5) Front Console.

The dispenser is provided with a particular console (Item 5 - Diagram 11), opening with a hinge, able to host in its interior:
- Personal Computer for controlling the mechanical pincer;
- Touch-screen monitor;
- Bar code reader for external products;

## Claims

1. Automatic dispensing unit for pharmaceutical packets comprising:
a first and a second storage unit (1, 4) comprising cells adapted to receive trays containing one or more of said pharmaceutical packets,
said second storage unit (4) having an exchange seale comprising a loading and unloading grid (3) for loading and unloading several of said trays into said first and second storage unit (1,4),
said loading and unloading grid (3) being open both to the outside and to the inside of the dispenser and being supplied with sensors to indicate presence of said pharmaceutical packets, the loading and unloading grid (3) comprising an electromechanical apparatus composed of a grid of several cells of various widths and lengths, able to accommodate pharmaceutical packets of varying dimensions placed on appropriate trays,
**characterized in that** the external opening of said loading and unloading grid (3) is fitted with an external door (6) which is served, mechanically and electronically, by an appropriate electric lock and an opening system with a powerful spring, and the inside opening of said loading and unloading grid (3) is provided with an horizontal sliding curtain (10), activated by an electric mechanism, which permits opening and closing to the inside of said loading and unloading grid (3) so as to prevent interferences with the inside of the dispenser and/or the accidental dropping of said trays inside the dispenser.

2. The automatic dispensing unit according to claim 1, **characterized in that** said exchange space (3) and said second storage unit (4) are arranged on a front side of said dispensing unit.

3. The automatic dispensing unit according to claims 1 or 2, **characterized in that** it further comprises a mechanical pincer (2) for presence detection, collection and transport of the trays containing the pharmaceutical packets, the mechanical pincer (2) takes the pharmaceutical packets from said loading and unloading grid (3) and inserts them into the free cells of said first and second storage unit (1, 4).

4. The automatic dispensing unit according to claim 3, **characterized in that** said mechanical pincer (2) is provided with a front sensor for detecting the presence of a tray, which permits scanning from low to high and vice versa of the whole of said loading and unloading grid (3).

5. The automatic dispensing unit according to one or more of the preceding claims, **characterized in that** it further comprises an active matrix system for reading the bar codes of said pharmaceutical packets.

6. The automatic dispensing unit according to claim 5, **characterized in that** said active matrix system reads the entire area of the tray and, detecting the presence of one or more of said packets, acquires an alpha-numeric string associated with the bar code.

7. The automatic dispensing unit according to claim 6, further comprising a software being adapted to interrogate with said acquired alpha-numeric string associated with the bar code an appropriate database containing identification of the products and additional information of the products.

8. The automatic dispensing unit according to one or more of the preceding claims, **characterized in that** the dispenser further comprises a single point (7) of withdrawal for individually withdrawing said packets by means of a conveyor belt with a sliding vertical control gate.

9. The automatic dispensing unit according to one or more of the preceding claims, **characterized in that** the dispenser further comprises a front console (5) housing inside it:
a Personal Computer for controlling the mechanical pincer (2);
a Touch-screen monitor (8);
a Bar code reader for external pharmaceutical packets;
a Belt for expulsion of single pharmaceutical packets.

10. The automatic dispensing unit according to claim 9, **characterized in that** said front console (5) houses inside it a reader (A) for recognition of finger prints.

11. The automatic dispensing unit according to one or more of the preceding claims, **characterized in that** said first and a second storage unit (1,4) comprise a metal internal storage unit subdivided into different types of cells for a total of approximately 800 cells.

## Patentansprüche

1. Automatische Abgabeeinheit für pharmazeutische Verpackungen, die Folgendes umfasst:
eine erste und eine zweite Lagereinheit (1, 4), die Zellen umfassen, welche ausgebildet sind, um Tabletts aufzunehmen, die eine oder mehrere der pharmazeutischen Verpackungen enthalten,
wobei die zweite Lagereinheit (4) einen Austauschraum hat, der ein Belade- und Entladegitter (3) zum Beladen und Entladen mehrerer der Tabletts in die erste und zweite Lagereinheit (1, 4) umfasst,
wobei das Belade- und Entladegitter (3) sowohl zur Außenseite als auch zur Innenseite der Abgabeeinheit hin offen ist und mit Sensoren versehen ist, um die Anwesenheit der pharmazeutischen Verpackungen anzuzeigen, wobei das Belade- und Entladegitter (3) eine elektromechanische Vorrichtung umfasst, die aus einem Gitter mehrerer Zellen verschiedener Breiten und Längen besteht, die fähig sind, pharmazeutische Verpackungen mit verschiedenen Maßen aufzunehmen, die auf entsprechenden Tabletts platziert sind,
**dadurch gekennzeichnet, dass** die äußere Öffnung des Belade- und Entladegitters (3) mit einer externen Tür (6) ausgestattet ist, die mechanisch und elektronisch von einem geeigneten elektrischen Schloss und einem Öffnungssystem mit einer starken Feder bedient wird, und wobei die innere Öffnung des Belade- und Entladegitters (3) mit einem horizontalen gleitenden Vorhang (10) versehen ist, der durch einen elektrischen Mechanismus aktiviert wird, der das öffnen und Schließen des Belade- und Entladegitters (3) zur Innenseite hin ermöglicht, um so Behinderungen mit der Innenseite der Abgabevorrichtung und/oder das zufällige Herunterfallen der Tabletts innerhalb der Abgabevorrichtung zu verhindern.

2. Die automatische Abgabeeinheit gemäß Anspruch 1, **dadurch gekennzeichnet, dass** der Austauschraum (3) und die zweite Lagereinheit (4) auf einer Vorderseite der Abgabeeinheit angeordnet sind.

3. Die automatische Abgabeeinheit gemäß Anspruch 1 oder 2, **dadurch gekennzeichnet, dass** sie weiter eine mechanische Zange (2) für die Anwesenheitserfassung, das Sammeln und den Transport der Tabletts umfasst, die die pharmazeutischen Verpackungen enthalten; wobei die mechanische Zange (2) die pharmazeutischen Verpackungen dem Belade- und Entladegitter (3) entnimmt und sie in die freien Zellen der ersten und zweiten Lagereinheit (1, 4) einsetzt.

4. Die automatische Abgabeeinheit gemäß Anspruch 3, **dadurch gekennzeichnet, dass** die mechanische Zange (2) mit einem vorderen Sensor zur Erfassung der Anwesenheit eines Tabletts ausgestattet ist, der ein Abtasten des gesamten Belade- und Entladegitters (3) von unten nach oben und umgekehrt ermöglicht.

5. Die automatische Abgabeeinheit gemäß einem oder mehreren der obigen Ansprüche, **dadurch gekennzeichnet, dass** sie weiter ein aktives Matrixsystem zum Lesen der Strichcodes der pharmazeutischen Verpackungen umfasst.

6. Die automatische Abgabeeinheit gemäß Anspruch 5, **dadurch gekennzeichnet, dass** das aktive Matrixsystem den gesamten Bereich des Tabletts liest und, indem es die Anwesenheit einer oder mehrerer der Verpackungen erfasst, eine alphanumerische Zeichenkette erfasst, die mit dem Strichcode verknüpft ist.

7. Die automatische Abgabeeinheit gemäß Anspruch 6, die weiter eine Software umfasst, welche ausgebildet ist, um mit der erfassten alphanumerischen Zeichenkette, die mit dem Strichcode verknüpft ist, eine entsprechende Datenbank abzufragen, die eine Identifikation der Produkte und zusätzliche Informationen zu den Produkten enthält.

8. Die automatische Abgabeeinheit gemäß einem oder mehreren der obigen Ansprüche, **dadurch gekennzeichnet, dass** die Abgabeeinheit weiter einen einzigen Entnahmepunkt (7) zur einzelnen Entnahme der Verpackungen mit Hilfe eines Förderbandes mit einem gleitenden vertikalen Steuertor umfasst.

9. Die automatische Abgabeeinheit gemäß einem oder mehreren der obigen Ansprüche, **dadurch gekennzeichnet, dass** die Abgabevorrichtung weiter eine vordere Konsole (5) umfasst, in der sich Folgendes befindet:
ein Personal Computer zur Steuerung der mechanischen Zange (2),
ein Touchscreen-Monitor (8),
ein Strichcode-Lesegerät für externe pharmazeutische Verpackungen,
ein Band zum Ausstoßen einzelner pharmazeutischer Verpackungen.

10. Die automatische Abgabeeinheit gemäß Anspruch 9, **dadurch gekennzeichnet, dass** sich in der vorderen Konsole (5) ein Lesegerät (A) zur Erkennung von Fingerabdrücken befindet.

11. Die automatische Abgabeeinheit gemäß einem oder mehreren der obigen Ansprüche, **dadurch gekennzeichnet, dass** die erste und eine zweite Lagereinheit (1, 4) eine interne Metall-Lagereinheit umfassen, die in verschiedene Arten von Zellen unterteilt ist, für insgesamt ungefähr 800 Zellen.

## Revendications

1. Unité de distribution automatique pour des confections pharmaceutiques comprenant :
une première et une seconde unités de stockage (1, 4) comprenant des cellules adaptées pour recevoir des plateaux contenant une ou plusieurs desdites confections pharmaceutiques,
ladite seconde unité de stockage (4) ayant un espace d'échange comprenant une grille de chargement et de déchargement (3) pour le chargement et le déchargement de plusieurs desdits plateaux dans ladite première et seconde unités de stockage (1, 4),
ladite grille de chargement et de déchargement (3) étant ouverte à la fois vers l'extérieur et l'intérieur du distributeur et étant pourvue de capteurs pour indiquer la présence desdites confections pharmaceutiques, la grille de chargement et de déchargement (3) comprenant un appareil électromécanique composé d'une grille de plusieurs cellules de diverses largeurs et longueurs, capable de loger des confections pharmaceutiques de dimensions variables placées sur des plateaux appropriés,
**caractérisée en ce que** l'ouverture externe de ladite grille de chargement et de déchargement (3) est dotée d'une porte externe (6) qui est desservie mécaniquement et électroniquement par un verrou électrique approprié et un système d'ouverture avec un ressort puissant, et l'ouverture interne de ladite grille de chargement et de déchargement (3) est dotée d'un rideau coulissant horizontal (10) activé par un mécanisme électrique qui permet l'ouverture et la fermeture vers l'intérieur de ladite grille de chargement et de déchargement (3) de sorte à éviter les interférences avec l'intérieur du distributeur et/ou l'affaissement accidentel desdits plateaux dans le distributeur.

2. Unité de distribution automatique selon la revendication 1, **caractérisée en ce que** ledit espace d'échange (3) et ladite seconde unité de stockage (4) sont disposés sur un côté avant de ladite unité de distribution.

3. Unité de distribution automatique selon la revendication 1 ou 2, **caractérisée en ce qu'**elle comprend en outre une pince mécanique (2) destinée à la détection de la présence, la collecte et le transport des plateaux contenant les confections pharmaceutiques, la pince mécanique (2) saisit les confections pharmaceutiques de ladite grille de chargement et de déchargement (3) et les insert dans les cellules libres de ladite première et seconde unités de stockage (1, 4).

4. Unité de distribution automatique selon la revendication 3, **caractérisée en ce que** ladite pince mécanique (2) est dotée d'un capteur avant pour la détection de la présence d'un plateau, qui permet le balayage du bas vers le haut et vice versa de l'ensemble de ladite grille de chargement et de déchargement (3).

5. Unité de distribution automatique selon l'une ou plusieurs quelconques des revendications précédentes, **caractérisée en ce qu'**elle comprend en outre un système de matrice active pour la lecture de codes-barres desdites confections pharmaceutiques.

6. Unité de distribution automatique selon la revendication 5, **caractérisée en ce que** ledit système de matrice active lit la zone entière du plateau, et en détectant la présence d'une ou plusieurs desdites confections, acquiert une chaîne alphanumérique associée au code-barres.

7. Unité de distribution automatique selon la revendication 6, comprenant en outre un logiciel adapté pour interroger avec ladite chaîne alphanumérique acquise associée au code-barres une base de données appropriée contenant l'identification des produits et des informations supplémentaires des produits.

8. Unité de distribution automatique selon l'une ou plusieurs quelconques des revendications précédentes, **caractérisée en ce que** le distributeur comprend en outre un seul point (7) de retrait pour le retrait individuel desdites confections à l'aide d'un convoyeur à courroie avec une porte de commande coulissant verticalement.

9. Unité de distribution automatique selon l'une ou plusieurs quelconques des revendications précédentes, **caractérisée en ce que** le distributeur comprend en outre une console avant (5) contenant :
un ordinateur personnel pour la commande de la pince mécanique (2) ;
un moniteur à écran tactile (8) ;
un lecteur de code-barres pour les confections pharmaceutiques externes ;
une courroie pour l'expulsion de confections pharmaceutiques individuelles.

10. Unité de distribution automatique selon la revendication 9, **caractérisée en ce que** ladite console avant (5) contient un lecteur (A) pour la reconnaissance d'empreintes digitales.

11. Unité de distribution automatique selon l'une ou plusieurs quelconques des revendications précédentes, **caractérisée en ce que** ladite première et une seconde unités de stockage (1, 4) comprennent une unité de stockage interne métallique subdivisée en différents types de cellules pour un total d'approximativement 800 cellules.
